# EUROPEAN PATENT SPECIFICATION

(11) **EP 1 541 159 B1**
(45) Date of publication and mention of the grant of the patent: **03.10.2012**
(21) Application number: 03028189.3
(22) Date of filing: 08.12.2003
(51) Int. Cl.: A61K 36/752, C07C 37/00

(54) **Antioxidant compositions of vegetal origin and process for their preparation**
Antioxidantien Zusammensetzung pflanzlicher Herkunft und Verfahren zur Herstellung
Composition antioxydative d'origine vegétale et procedure de sa préparation

(43) Date of publication of application: 15.06.2005
(73) Proprietor: CHEVALLEY, Isabelle, 1188 ST-GEORGE (CH)
(72) Inventor: CHEVALLEY, Isabelle, 1188 ST-GEORGE (CH)
(74) Representative: Vuille, Roman

(56) References cited:
- EP-A- 1 317 928
- RU-C- 2 177 793
- US-A- 5 928 646
- US-B2- 6 506 421

## Description

### Field of the invention

The invention refers to the valorisation of vegetal waste material, and to antioxidant compositions which can be gained there from and which can be used in food, cosmetic or pharmaceutical industry.

### Background of the invention

Antioxidants have been widely used since decades, e.g. as stabilisers for foods, beverages or cosmetic products, or even in synthetic materials like polymers sensitive to oxygen or light during long term storage. They have also been used for their specific metabolic properties, most frequently as nutritional supplements like vitamins or as medicaments like many free radical scavengers.

Antioxidants are frequently met as pure chemical entities of either chemical or natural origin such as e.g. BHA, BHT, vitamins C or E. They also exist in the form of more or less complex mixtures such as purified essential oils, e.g. rosmarine oil or sage oil, or e.g. as mixtures of polyphenols.

The current trend in that field is to diversify as broadly possible the use as well as the origin of antioxidants in order to meet new requirements like their compatibility to specific food, cosmetic or pharmaceutical ingredients or their compatibility towards specific processing pathways. Also, antioxidant ingredients or antioxidant compositions are facing today increasingly stringent food regulations, in terms of safety or commercial constraints like e.g. yield or manufacture price.

Vegetal raw materials or vegetal residues have been already proposed and used as a source of useful products e.g. sweeteners, food additives or colorants like hesperidin. - see e.g. ES 86 04 919A1; ES 86 03 907A1; ES 59 1268 A5; JP 81 88593; JP 62 11624.

WO01/78859 discloses a method for extracting valuable material like pectines or polyphenols from fruit or vegetable waste material and namely vegetable or fruit pressing residue. Said waste material is first subject to acidic treatment, then filtered and concentrated to afford a so called "pectin extract" which is subsequently either subject to an enzymatic treatment or simply poured onto a separation column.

US 5 994 413 discloses a method for producing fruit polyphenols from unripe fruits of Rosaceae subject to pressing or extraction, wherein unripe apple juice is added with pectolyis enzyme, filtered passed through a resin column for removing the saccharides.

WO98/11789 discloses a method for preparing a flavonols containing composition derived from wine. The problem at stake here consists to extract the flavonols from wine and subsequently purify them adequately.

Antioxidants of vegetal origin have the advantage to meet with the "natural trend" of the current century and also, more objectively, to propose natural antioxidant mixtures or compositions which are at first glance food safe and which can easily cope with the current regulatory requirements.

When considering another aspect typical of the problem, one could assume that subjecting materials such as fruit pressing residues to an extraction leading to antioxidants would add significant value to the entire process, i.e. the juice manufacturing process.

The purpose of the invention is to offer new, original and highly performing antioxidant compositions and also to propose a new and efficient way to valorise current industrial processes as those met e.g. in food and beverage industry.

### The invention

These objectives are met by means of the present invention

The invention refers to a method for the valorisation of vegetal waste material which comprises subjecting pre-processed material of vegetal origin to a series of processing steps leading, on one hand, to a composition consisting essentially of mono-, di or polysaccharides and, on the other hand, to a compositions consisting essentially of antioxidants substantially free of polysaccharides, the said mono-, di and polysaccharide composition being further converted into energetic substrate, like e.g. alcohol or biogas.

### Detailed description of the invention

According to the invention, the antioxidant composition can be prepared by
a) contacting pre-processed material of vegetal origin with an organic polar solvent and performing agitation of the mixture until saturation of the organic polar solvent in extractible substances;
b) drying off the organic solution afforded according to step a);
c) adding water to the dried material until complete dissolution of the extracted material;
d) passing the aqueous solution thus afforded onto a polymer resin material suitable for fixing selectively polyphenols or the like while allowing the selective elution of mono- , di- or polysaccharides;
e) eluting the whole mass with water until complete migration of the mono-, di- and polysaccharides;
f) eluting the remaining antioxidant substances fixed to the polymer resin with a low molecular weight aliphatic alcohol; and eventually
g) drying off the eluted mixture.

Pre-processed material covers any stuff or mixture deemed to contain antioxidant substances in reasonable amount, e.g. fruits and vegetable pressing residues as afforded in juice manufactures, or any fruit or vegetable source which has been processed in any food industry, e.g. residues of jam, marmalade or jelly manufacture, or of peeled and portioned or sliced fruits or vegetables sold in cans.

Most conveniently the vegetal material is selected form fruits like citrus, tomatoes, pineapples, apples, pears, grapes, berries or oleaginous fruits like olive, almonds, nuts or from vegetables like broccoli's, carrots, celery, parsley or even water plants, algae or phytoplankton although such as list is not exhaustive.

A great advantage of the present invention is to provide a process which can make direct, i.e. in line use of freshly pressed vegetal material or residue as immediately achievable from the juice pressing operations, without superfluous or expensive transportation or storage operations. Thus only a mere extension of existing industrial lines will be necessary to get a valorisation of the current juice manufacture process. The man skilled in the art will be able to detect additional industrial or commercial advantages easily.

When the starting vegetal material contains or is deemed to contain lipophilic substances like terpenes, mineral oils, waxes or even contaminants as frequently met in agriculture said material is first extracted by means of a lipophilic organic solvent prior to the treatment of step a) here above.

As organic lipophilic solvent one can use an aliphatic hydrocarbon such as hexane or a mixture of aliphatic hydrocarbons such as petrol ether, preferably of food grad qualities in order to avoid any additional contamination of the matter which will be then subjected to the process of the present invention.

Whether subjected or not to the previous treatment with a lipophilic extraction solvent mentioned here above, the pre-processed material is contacted with an organic polar solvent as referred to in step a) above. The said organic polar solvent is selected from aliphatic alcohols, aliphatic esters or ethers, preferably from food grade aliphatic alcohols like ethanol.

The subsequent drying step is performed using common techniques like vacuum evaporation, which has the advantage not to necessitate excessive heating, usually 40° C max., and consequently to keep intact heat sensitive antioxidant substances. Due to the presence of many polysaccharides or similar polar compounds in the organic extract, there is obtained a humid and sticky mass which cannot be easily further handled or processed as is. Its antioxidant power is relatively low moreover due to the high proportion of ballast material still present therein.

A crucial feature of the process of the invention consists to treat said mass according to steps c) to e) set forth here above.

Usually water is added to the mass resulting from drying step b) until the achievement of a limpid, somewhat coloured solution. This represents the easiest reference method to implement for control as the nature of the above organic extract varies extensively depending on the origin or on the variability of the fruits or vegetables subject to pre-processing. So the man skilled in the art would have to adjust the proportions of relevant components of the reaction mixture, but this can be performed without difficulties.

The limpid aqueous solution is then transferred onto a polymer resin material suitable for fixing selectively mono-, di- or polysaccharides and also, whenever present, other hydroxyl-compounds such as high molecular weight alcohols, hydroxy-acids or hydroxyesters. Such resins are commercially available. Good results have been achieved making use of DIAION ® (MITSUBISHI CHEMICAL) e.g. with the type resins currently proposed for industrial purposes.

The resin column is then eluted with water, an operation which induces the progressive migration of said saccharides or hydroxy-compounds, whereas the antioxidant substances remain almost immobilized. The selective elution is easy to control as the antioxidant mixture comprises frequently yellow coloured substances, namely polyphenols and more specifically flavonoïds. The presence of polysaccharides in the water phase can be controlled by chromatography or any equivalent tracing methods. Once said elution is complete the aqueous mixture is set aside.

According to one embodiment of the invention the aqueous polysaccharides mixture thus afforded is advantageously and cheaply converted into an energetic substrate like e.g. low molecular weight aliphatic alcohols or esters, methanol or ethanol (green gasoline) or into biogas. This conversion can be achieved according to the current technology like e.g. fermentation by means of suitable yeasts or bacteria or by enzymatic degradation. Advantageously said fermentation treatment can be performed on the aqueous mixture as directly achieved according to the invention (see above) or after concentration up to the desired dry matter content in polysaccharide.

The elution of the remaining fraction, i.e. the one comprising antioxidant substances like e.g. polyphenols and flavonoïds is then performed by means of a low molecular weight aliphatic alcohol. Within the frame of the present invention methanol and ethanol proved most efficient in many instances. Complete exhaustion of the polymer resin is easily followed by visual control as most of the antioxidants present in vegetal material are coloured substances, like e.g. flavonoïds. More elaborated detection methods can also be used therefore. Eventually, the alcoholic eluate is evaporated up to complete dryness, usually under reduced pressure and moderate heating to afford in most instances a yellow powdered mass.

The analysis of the powder mass so achieved has shown that, depending on the fruit or vegetable source used according to the invention, this powder consists of a more or less complex mixture of polar substances present in variable proportions like phenolic compounds and flavonoids such as those listed here after concerning citrus fruits or apples : hesperidine, isosakuranetine rutinoside, narirutine, eriocitrine, tangeretin, sinensetine, tetra-O-mehtylscutellareine, nobiletine, hexa-O-methylgossypetine, hexa-O-methylquercetagetine, heptamethoxyflavone, 5-hydroxy-3,7,8,3',4'-pentmethoxyflavone, quercetine, quercitrin, isoquercitrine, rutine, hyperoside, reynoutine, guaijavernie, avicularin, apigenine, luteoline, catechine, dihydroquercitine, cyanidine, malvidine, etc. Evidently, the above list is depicting only some of the compounds which can be present in an antioxidant composition according to the invention.

The antioxidant composition resulting from the process mentioned here above exhibits a strong antioxidant activity when tested e.g. *in vitro* against DPPH or *in vivo* by means the KRL Spiral test (see in this respect US 5,135,850 of 04.08.1992). Due to that particular feature it can be used conveniently either in the form of a raw extract, a purified extract, a solution, a powder or in the form of coated or encapsulated particles, for example as encapsulated food supplements or food complements. When considered food additives, they are usually incorporated to the relevant foods, beverages, cosmetic or pharmaceutical products in proportions which are comprised between about 0.1 % and about 10.0 % weight although these values are not considered as absolute limits.

In such foods or beverages, cosmetic or pharmaceutical products, the composition according to the invention can be used either as the unique antioxidant or as a co-ingredient, in admixture with usual antioxidant molecules.

The examples here below merely illustrate the present invention.

### Example 1

### Antioxidant composition extracted from orange peels

About 2 kg of orange peels as resulting from a pressing operation have been shredded into smaller pieces and then extracted under agitation (24 hours; room temperature) with 4 1 of hexane.

The extracted peel mass has been then mixed with about twice its weight of ethanol and then kept under agitation for 24 hours at room temperature and the alcoholic extract eventually subjected to solvent evaporation under vacuum, at max. 40°C.

There was obtained a sticky yellowish humid mass which was then immediately diluted with water until production of a limpid colloidal suspension. Then samples of ca 20g of evaporated ethanol extract were diluted with 25 ml of water and these portions poured onto a column having a volume of about 250 ml and filled with DIAION ® HP20L resin (Mitsubishi Chemical; ca 100 g of resin), then eluted with 1000 ml of water and subsequently eluted with 750 ml methanol. The aqueous eluate contains various saccharides like fructose, saccharose, galactose, mannitose and inositol as confirmed by TLC. It has been set aside as is.

The methanol extract (or eluate) was eventually evaporated under vacuum (temp. max. 40°C) to dryness to afford 1.58 g of a yellow powder which has been then tested by TLC against prior known antioxidant flavonoïds (reference compounds were selected among those available in current analytical laboratories, e.g. among those listed previously); the presence of antioxidant flavonoïds in the above dry material has been confirmed.

### Example 2

### Orange peels used as source of antioxidants; variants

The process of example I has been reproduced using the same starting "defatted" raw material. Similar extraction results have been achieved when substituting ethanol with ethyl acetate alone, or various mixtures of e.g. ethyl acetate, although final yield was lower.

### Example 3

### Antioxidant composition extracted from apple pulp

About 1.240 kg of apple residue material resulting from a pressing operation has been mixed with about twice its weight of ethanol and then kept under agitation for 24 hours at room temperature, then filtered and the alcoholic extract eventually subjected to solvent evaporation by means of lyophilisation to afford ca 50 g of extract.

There was obtained a sticky light yellowish humid mass which was then immediately diluted with water until production of a limpid colloidal suspension. The suspension thus achieved was poured onto a column having a volume of about 250 ml and filled with DIAION ® HP20L resin (Mitsubishi Chemical; ca 100 g of resin), then eluted with 2000 ml of water and subsequently eluted with 3000 ml ethanol. The aqueous eluate which contains various saccharides has been set aside as is.

The ethanol extract (or eluate) was eventually evaporated under vacuum (temp. max. 40°C) to dryness to afford 2.58 g of a light yellow powder which has been then tested by TLC against prior known antioxidant (reference compounds were selected among those available in current analytical laboratories, e.g. among those listed previously); the presence of antioxidant polyphenols or flavonoïds in the above dry material has been confirmed.

### Example 4

### Confirmation of the antioxidant activity of the polyphenols extracts

4.1 DPPH tests have been performed according to the current method using samples of the "orange extract" manufactured according to example 1 and samples of the "apple extract" prepared according to example 3. Reference material comprised aqueous extracts (infusions) of green tea and dog rose, respectively. Corresponding results are reported here below wherein concentration values are given in mg of dry material per 1 and the results are given in % of antioxidant activity.

| Concentration (mg/l) | orange extract | apple extract | green tea extract | Dog rose extract |
|---|---|---|---|---|
| 1.10 | 1.60 | 1.20 | 4.80 | 8.10 |
| 2.23 | 5.30 | 2.10 | 6.70 | 8.70 |
| 3.45 | 11.70 | 3.00 | 8.40 | 12.70 |
| 8.90 | 11.20 | 3.80 | 9.60 | 15.30 |
| 17.80 | 17.10 | 31.60 | 19.90 | 24.10 |

This confirms that the process of the invention does not affect the antioxidant activity of the compounds so afforded; it also show that "apple extract" expresses a significantly high antioxidant power as compared to the reference compounds, at relatively high concentrations.

4.2 Spiral KRL tests (US Pat 5,135,850 - "Process for the determination by means of free radicals of the antioxidant properties of a living organism ...") have been performed according to the currently certified method using samples of the "orange extract" manufactured according to example 1 and samples of the "apple extract" prepared according to example 3. Reference material comprised aqueous extracts (infusions) of green tea and dog rose, respectively. Corresponding results are reported here below wherein concentration values are given in mg of dry material per 1 and the results are given in % of antioxidant activity.

| Concentration (mg/l) | orange extract | apple extract | green tea extract | Dog rose extract |
|---|---|---|---|---|
| 10.0 | 66.9 | 67.4 | 43.6 | 22.4 |
| 20.0 | 103.6 | 134.2 | 107.4 | 42.9 |
| 50.0 | 198.9 | 294.1 | 302.8 | 117.0 |
| 100 | 305.8 | 409.8 | 205.7 | 235.9 |

This further confirms that the process of the invention does not affect the antioxidant activity of the compounds so afforded; it also shows that "orange extract" and "apple extract" as well expresses a significantly strong antioxidant power at concentrations as high as 100 mg/l whereas reference green tea, at the same concentration, extract progressively affects the red blood cells used for such a testing.

## Claims

1. A method for the valorisation of pre-processed vegetal waste material selected from freshly pressed vegetal material or residue as directly achievable from juice pressing operations which comprises subjecting the pre-processed material to a series of processing steps comprising:
a) contacting pre-processed material of vegetal origin with an organic polar solvent and performing agitation of the mixture until saturation of the organic polar solvent in extractible substances;
b) drying off the organic solution afforded according to step a);
c) adding water to the dried material until complete dissolution of the extracted material;
d) passing the aqueous solution thus afforded onto a polymer resin for fixing selectively the polyphenols while allowing selective elution of mono-, di- or polysaccharides; and
e) eluting the whole mass with water until complete migration of the mono-, di- and polysaccharides, the polysaccharide composition being further converted into an energetic substrate, like e.g. alcohol or biogas.

2. The method according to claim 1 which comprises further eluting the remaining polyphenols with a low molecular weight aliphatic alcohol and drying of the eluted alcoholic mixture to afford an antioxidant composition substantially free of mono-, di- and polysaccharides.

3. The method according to claim 1 wherein the pre-processed vegetal material is freshly pressed vegetal material or residue of fruits like citrus, tomatoes, pineapples, apples, pears or grapes.

4. The method according to claim2 1 to 3 wherein, when the pre-processed material contains lipophilic components, it is first extracted by means of a lipophilic organic solvent prior to the treatment of step a) here above.

5. The process according to any of claims 1 to 4 wherein the organic polar solvent is selected from aliphatic alcohols, aliphatic esters or ethers, preferably from food grade aliphatic alcohols like ethanol or methanol.

## Patentansprüche

1. Ein Verfahren zur Verwertung von vorverarbeitetem Pflanzenabfallmaterial ausgewählt aus frisch gepresstem Pflanzenmaterial oder Rückstände, wie sie direkt aus Saftpressverfahren erhalten werden können, umfassend das Unterwerfen des vorverarbeiteten Materials einer Reihe von Behandlungsschritten, die umfassen:
a) das in Kontaktbringen des vorverarbeiteten Materials pflanzlicher Herkunft mit einem organischen, polaren Lösungsmittel und das Durchführen einer Durchmischung bis zur Saturierung des organischen Lösungsmittels von extrahierbaren Substanzen;
b) das Abdampfen der in Schritt a) zubereiteten organischen Lösung;
c) das Hinzufügen von Wasser zum getrockneten Material bis zur kompletten Auflösung des extrahierten Materials;
d) das Weiterleiten der so erhaltenen wässrigen Lösung auf ein Polymerharz um selektiv Polyphenole zu fixieren, während das selektive Ausspülen von Mono-, Di- und Polysacchariden erlaubt wird;
e) das Ausspülen der gesamten Masse mit Wasser bis zur kompletten Migration der Mono-, Di- und Polysaccharide, wobei die Polysaccharidzusammensetzung anschliessend zu einem Energieträger wie zum Beispiel Alkohol oder Biogas konvertiert wird.

2. Das Verfahren gemäss Anspruch 1, ferner umfassend das Ausspülen der verbleibenden Polyphenole mit einem aliphatischen Alkohol niedrigen Molekulargewichts und das Trocknen der ausgespülten alkoholischen Mischung um eine Antioxidanszusammensetzung zu erhalten, die im Wesentlichen frei von Mono-, Di- und Polysacchariden ist.

3. Das Verfahren gemäss Anspruch 1, wobei das vorverarbeitete Pflanzenmaterial frisch gepresstes Pflanzenmaterial oder ein Rückstand von Früchten wie Zitrusfrüchten, Tomaten, Ananas, Äpfeln, Birnen oder Trauben ist.

4. Das Verfahren gemäss der Ansprüche 1 bis 3, wobei das vorbehandelte Material, wenn es lipophile Komponenten umfasst, vor der Behandlung gemäss obigem Schritt a) mittels einem lipophilen organischen Lösungsmittel extrahiert wird.

5. Das Verfahren gemäss einem beliebigen der vorangehenden Ansprüche 1 bis 4, wobei das organische polare Lösungsmittel von aliphatischen Alkoholen, aliphatischen Estern oder Ethern, vorzugsweise von lebensmitteltauglichen aliphatischen Alkoholen wie Ethanol oder Methanol ausgewählt wird.

## Revendications

1. Un procédé pour la valorisation de déchets de matériau végétal prétraité choisis parmi le matériau végétal fraîchement pressé ou le résidu obtenu directement des opérations de pressage de jus, qui comprend de soumettre le matériau prétraité à une série d'étapes de procédé comprenant :
a) mettre en contact du matériau prétraité d'origine végétale avec un solvant organique polaire et effectuer l'agitation du mélange jusqu'à saturation du solvant organique polaire en substances extractibles ;
b) évaporer à sec la solution organique obtenue selon l'étape a) ;
c) ajouter de l'eau au matériau séché jusqu'à dissolution complète du matériau extrait ;
d) faire passer la solution aqueuse ainsi obtenue sur résine polymère pour fixer sélectivement les polyphénols tout en autorisant l'élution des mono, di ou polysaccharides ; et
e) éluer la masse avec de l'eau jusqu'à migration complète des mono, di ou polysaccarides, la composition de polysaccharides étant ensuite convertie en un substrat énergétique tel que par exemple alcool ou biogaz.

2. Le procédé selon la revendication 1 qui comprend ensuite l'élution des polyphénols subsistants avec un alcool aliphatique de bas poids moléculaire et l'évaporation à sec du mélange alcoolique élué pour obtenir une composition antioxydante essentiellement débarrassée de mono, di ou polysaccharides.

3. Le procédé selon la revendication 1 dans lequel le matériau végétal prétraité est un matériau végétal fraîchement pressé ou un résidu de fruits tels qu'agrumes, tomates, ananas, pommes, poires ou raisins.

4. Le procédé selon les revendications 1 à 3 dans lequel, lorsque le matériau prétraité contient des composants lipophiles, il est premièrement extrait au moyen d'un solvant organique lipophile, préalablement au traitement selon l'étape a) ci-dessus.

5. Le procédé selon l'une des revendications 1 à 4 dans lequel le solvant organique polaire est choisi parmi les alcools aliphatiques, les esters ou les éthers aliphatiques, de préférence les alcools aliphatiques « food grade » tels qu'éthanol ou méthanol.
